# EUROPEAN PATENT APPLICATION

(11) **EP 3 903 740 A1**
(43) Date of publication of application: **03.11.2021**
(21) Application number: 19902886.1
(22) Date of filing: 31.10.2019
(51) Int. Cl.: A61F 2/82

(54) **DRUG ELUTING INSTRUMENT AND PREPARATION METHOD THEREFOR**

(30) Priority: 27.12.2018 CN 201811615405
(71) Applicant: Biotyx Medical (Shenzhen) Co., Ltd., Shenzhen, Guangdong 518000 (CN)
(72) Inventor: CHEN, Liping, Shenzhen, Guangdong 518000 (CN); LIN, Wenjiao, Shenzhen, Guangdong 518000 (CN); ZHANG, Wanqian, Shenzhen, Guangdong 518000 (CN)
(74) Representative: Prinz & Partner mbB
(86) International application number: PCT/CN2019/114698
(87) International publication number: WO 2020/134541

(57) **Abstract**

A drug eluting device and a preparation method thereof are disclosed. The drug eluting device includes a substrate and a drug coating arranged on the substrate. The substrate has an outer wall, side walls, and an inner wall, the drug coating contains active drug and a drug carrier, the active drug is dispersed in the drug carrier, the drug coating covers the outer wall, the side walls, and the inner wall of the substrate, and the content of the active drug on both the outer wall and the side walls are greater than the content on the inner wall; or, the active drug is only distributed on the outer wall and the side walls, and the inner wall does not contain the active drug. The drug eluting device is less prone to the phenomenon of coating falling from the substrate.

## Description

### Field

The present disclosure relates to the field of interventional medical devices, and particularly relates to a drug eluting device and a preparation method thereof.

### Background

A vascular stent is an endovascular implant with expandability and can play a role in radial supporting after being implanted into a diseased vascular site to achieve revascularization. A drug eluting stent is generally characterized in that the surface of a bare stent substrate is coated with a drug coating, the drug coating contains an active drug, and the occurrence of vascular restenosis is inhibited through the release of the active drug.

After the drug eluting stent is implanted into a blood vessel, only the outer wall and side walls of the stent are in contact with the vessel wall, the active drug released from the outer wall and side walls of the stent enters the vessel wall to inhibit intimal hyperplasia of the vessel wall and prevent restenosis of the blood vessel, while the active drug on the inner wall of the stent cannot inhibit intimal hyperplasia but is released into blood to circulate to the whole body to produce side effects on other organs. In addition, the active drug on the inner wall of the stent further inhibits stent endothelialization, thereby increasing the risk of thrombosis.

In order to solve the above problems, the existing drug eluting stent is only provided with a drug coating on the outer wall and the side walls, and the drug coating or any other coating is not provided on the inner wall of the stent.

However, with regard to a balloon-expanded stent, stent rods are significantly deformed from a compressed state to an expanded state of the stent, and a coating on the stent is also significantly deformed. Therefore, in the case of the stent with the coating on the outer wall and the side walls and without the coating on the inner wall, peeling between the coating and the stent substrate may occur when the stent is expanded. When the coating is incomplete, the risk of peeling of the coating is exacerbated, which may result in falling of the coating from the substrate into the blood, thereby increasing the risk of embolisms and thrombi.

### Summary

In view of this, the present disclosure provides a drug eluting device, the inner wall of which does not contain an active drug or contains less active drug, and which is less prone to the phenomenon of coating falling from the substrate.

A drug eluting device includes a substrate and a drug coating arranged on the substrate, the substrate has an outer wall, side walls, and an inner wall, the drug coating contains active drug and a drug carrier, the active drug is dispersed in the drug carrier, the drug coating wraps the outer wall, the side walls, and the inner wall of the substrate, and both of the content of the active drug on the outer wall and the content of the active drug on the side walls are greater than the content of the active drug on the inner wall; or, the active drug is only distributed on the outer wall and the side walls, and the inner wall does not contain the active drug.

In an embodiment, a portion of the drug coating on the inner wall has a pore structure.

In an embodiment, the pore structure has a porosity is in the range of 5% to 60%.

In an embodiment, in the drug coating, the content of the active drug is in the range of 0.5 µg/mm² to 6 µg/mm².

In an embodiment, the drug carrier is a degradable polymer, and the degradable polymer is selected from at least one of a degradable polyester and a degradable anhydride.

In an embodiment, the drug coating has a thickness in the range of 2 microns to 20 microns.

In an embodiment, both of the thickness of a portion of the drug coating on the outer wall and the thickness of a portion of the drug coating on the side walls are greater than the thickness of the portion of the drug coating on the inner wall.

In an embodiment, the material of the substrate is pure iron or iron alloy.

In an embodiment, the drug eluting device further includes a zinc-containing protective layer, the zinc-containing protective layer is arranged on the substrate, the zinc-containing protective layer covers at least the inner wall of the substrate, and the zinc-containing protective layer is covered by the drug coating.

In an embodiment, the coverage of the zinc-containing protective layer on the substrate is in the range of 20%-100%.

A preparation method of a drug eluting device includes the following steps:
providing a substrate, the substrate having an outer wall, side walls, and an inner wall;
applying a solution containing an active drug and a drug carrier to the substrate to form a drug coating, wherein the active drug is dispersed in the drug carrier, and the drug coating covers the outer wall, the side walls, and the inner wall of the substrate; and
using a solvent to contact a portion of the drug coating on the inner wall such that the active drug at the portion of the drug coating on the inner wall is partially or completely dissolved in the solvent and the drug carrier is insoluble in the solvent, and removing the solvent from the substrate.

In an embodiment, the step of using the solvent to contact the portion of the drug coating on the inner wall such that the active drug at the portion of the drug coating on the inner wall is partially or completely dissolved in the solvent and the drug carrier is insoluble in the solvent, and removing the solvent from the substrate includes using an adsorbent material adsorbing the solvent to contact the inner wall of the substrate such that the active drug at the portion of the drug coating on the inner wall is partially or completely dissolved in the solvent and the drug carrier is insoluble in the solvent, and then removing the adsorbent material.

In an embodiment, the adsorbent material is a nylon material, polypropylene, a polytetrafluoroethylene material, a rubber-like material, or a fiber-like material.

In an embodiment, the time of contact between the adsorbent material adsorbing the solvent and the inner wall of the substrate is in the range of 10 s to 20 min.

In an embodiment, the step of using the adsorbent material adsorbing the solvent to contact the inner wall of the substrate such that the active drug at the portion of the drug coating on the inner wall is partially or completely dissolved in the solvent and the drug carrier is insoluble in the solvent, and removing the adsorbent material includes:
providing a core shaft, wrapping the outer surface of the core shaft with the adsorbent material, then putting the core shaft wrapped with the adsorbent material into the interior of the substrate, causing the adsorbent material to adsorb the solvent, using the adsorbent material to contact the inner wall of the substrate such that the active drug at the portion of the drug coating on the inner wall is partially or completely dissolved in the solvent, and then removing the adsorbent material and the core shaft from the substrate; or,
providing a core shaft, wrapping the outer surface of the core shaft with the adsorbent material, causing the adsorbent material to adsorb the solvent, then putting the core shaft wrapped with the adsorbent material into the interior of the substrate, using the adsorbent material to contact the inner wall of the substrate such that the active drug at the portion of the drug coating on the inner wall is partially or completely dissolved in the solvent, and then removing the adsorbent material and the core shaft from the substrate; or,
providing a core shaft made of the adsorbent material, causing the core shaft to adsorb the solvent, then putting the core shaft adsorbing the solvent into the interior of the substrate, using the core shaft to contact the inner wall of the substrate such that the active drug at the portion of the drug coating on the inner wall is partially or completely dissolved in the solvent, and then removing the core shaft from the substrate; or,
providing a core shaft made of the adsorbent material, putting the core shaft into the interior of the substrate, causing the core shaft to adsorb the solvent, using the core shaft to contact the inner wall of the substrate such that the active drug at the portion of the drug coating on the inner wall is partially or completely dissolved in the solvent, and then removing the core shaft from the substrate; or,
wiping the inner wall of the substrate with the absorbent material absorbing the solvent such that the active drug at the portion of the drug coating on the inner wall is partially or completely dissolved in the solvent, and then removing the adsorbent material from the substrate.

In an embodiment, in the process of contact between the adsorbent material adsorbing the solvent and the inner wall of the substrate, the core shaft or the substrate is in a rotated state.

In an embodiment, after the step of using the solvent to contact the portion of the drug coating on the inner wall such that the active drug at the portion of the drug coating on the inner wall is partially or completely dissolved in the solvent and the drug carrier is insoluble in the solvent, and removing the solvent from the substrate, the content of the active drug on the substrate is reduced by up to 50%.

Regarding the drug coating of the drug eluting device, the distribution of the active drug in the drug coating satisfies the condition that both of the content of the active drug on the outer wall and the content of the active drug on the side walls are greater than the content the active drug on the inner wall or the active drug is only distributed on the outer wall and the side walls and the portion of the drug coating on the inner wall does not contain the active drug, and the drug coating coats the outer wall, the side walls and the inner wall of the substrate, i.e. the drug coating is a complete and continuous coating, so that during expansion, the drug coating does not readily separate from the substrate and fall off from the substrate. Therefore, the inner wall of the drug eluting device does not contain the active drug or the content of the active drug on the inner wall is low, and the drug eluting device is less prone to the phenomenon of coating falling.

### Brief Description of the Drawings

Fig. 1 is a cross-sectional structure diagram of a drug eluting device according to an embodiment.
Fig. 2 is a cross-sectional structure diagram of a drug eluting device according to another embodiment.
Fig. 3 is a cross-sectional structure diagram of a drug eluting device according to another embodiment.
Fig. 4 is a cross-sectional structure diagram of a drug eluting device according to another embodiment.

### Detailed Description

In order to make the above objects, features and advantages of the present disclosure easier to understand, specific embodiments of the present disclosure will be described in detail below in conjunction with the accompanying drawings. In the following description, numerous specific details are illustrated for full understanding of the present disclosure. However, the present disclosure can be implemented by other means different from those described here, a person skilled in the art may make similar improvements without departing from the essence of the present disclosure, and therefore, the present disclosure is not limited to the specific embodiments disclosed herein.

Unless otherwise defined, all technical and scientific terms used herein have the same meanings as commonly understood by those of ordinary skill in the technical field of the present disclosure. The terms used in the description of the present disclosure are for the purpose of describing specific embodiments, but are not intended to limit the present disclosure.

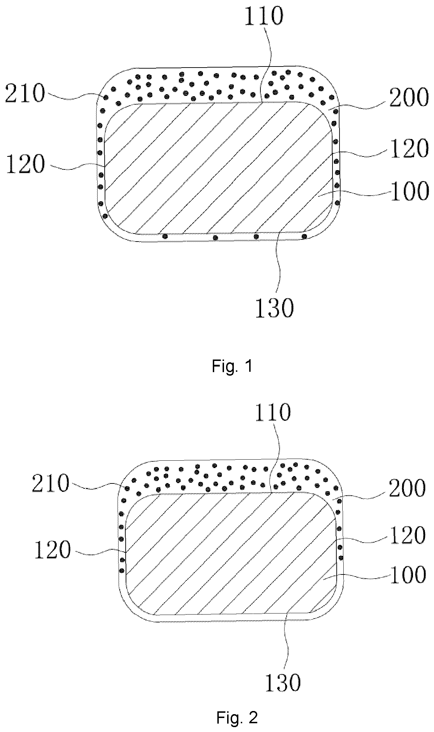

Referring to Fig. 1, a drug eluting device according to an embodiment includes a substrate 100 and a drug coating 200 arranged on the substrate 100.

The substrate 100 is of a hollowed lumen structure. The substrate 100 has an outer wall 110, side walls 120 and an inner wall 130.

In an embodiment, the substrate 100 is made of a bioabsorbable material. For example, the substrate 100 is made of iron, an iron-based alloy, magnesium, a magnesium-based alloy, or an absorbable polymer material. In an embodiment, the substrate 100 is made of an iron-based alloy having a carbon content of not more than 2.11 wt.% or the substrate 100 is made of pure iron.

In other embodiments, the substrate 100 is made of a non-bioabsorbable material. For example, the substrate 100 is made of a nickel-titanium alloy, a cobalt-chromium alloy, or stainless steel.

The drug coating 200 coats the outer wall 110, the side walls 120 and the inner wall 130 of the substrate 100, i.e., the drug coating 200 is a complete and continuous coating, and all surfaces of the substrate 100 are covered by the drug coating 200.

The drug coating 200 contains an active drug 210 and a drug carrier (not shown), the active drug 210 is non-uniformly dispersed in the drug carrier, i.e., the distribution of the active drug 210 in the drug coating 200 is non-uniform distribution. In an embodiment, as shown in Fig. 1, both of the content of the active drug 210 on the outer wall 110 and the content of the active drug 210 on the side walls 120 are greater than the content of the active drug 210 on the inner wall 130. Specifically, the content per unit area of the active drug 210 on the outer wall 110 and the content per unit area of the active drug 210 on the side walls 120 are greater than the content per unit area of the active drug 210 on the inner wall 130.

In another embodiment, as shown in Fig. 2, the active drug 210 is only distributed on portions of the drug coating 200 on the outer wall 110 and the side walls 120, and a portion of the drug coating 200 on the inner wall 130 does not contain the active drug 210.

Regarding the drug coating 200 of the drug eluting device, the distribution of the active drug 210 in the drug coating 200 satisfies the condition that both of the content of the active drug 210 on the outer wall 110 and the content of the active drug 210 on the side walls 120 are greater than the content of the active drug 210 on the inner wall 130, or the active drug 210 is only distributed on the outer wall 110 and the side walls 120, the inner wall 130 does not contain the active drug 210, and the drug coating 200 coats the outer wall 110, the side walls 120 and the inner wall 130 of the substrate 100, i.e., the drug coating 200 is a complete and continuous coating, so that during expansion, the drug coating 200 does not readily separate from the substrate 100 to fall off from the substrate 100. Therefore, the inner wall 130 of the drug eluting device does not contain the active drug 210 or the content of the active drug 210 on the inner wall 130 is low, and the drug eluting device is less prone to the phenomenon of coating falling.

Therefore, because the inner wall 130 of the substrate 100 does not contain the active drug 210 or the content of the active drug 210 on the inner wall 130 is low, rapid endothelialization of the drug eluting device is facilitated, and the risk of thrombosis is reduced. In addition, the drug released into the blood is reduced, thereby reducing the toxic and side effects on other organs and improving the safety of clinical use.

In an embodiment, the active drug 210 is selected from at least one of a cytostatic agent, a corticoid, a prostacyclin, an antibiotic, a cell proliferation inhibitor, an immunosuppressor, an anti-inflammatory agent, an antiphlogistic, an anti-angiogenic drug, an anti-restenosis drug, an anti-thrombotic drug, an anti-allergic drug, and an anti-tumor drug. In an embodiment, the drug that inhibits vascular proliferation is selected from at least one of paclitaxel, rapamycin and derivatives thereof. The anti-platelet drug is cilostazol. The anti-thrombotic drug is heparin. The anti-inflammatory drug is dexamethasone. The anti-allergic drug is selected from at least one of calcium gluconate, chlorpheniramine maleate and cortisone.

In an embodiment, in the drug coating 200, the content of the active drug 210 is 0.5 µg/mm² to 6 µg/mm². It should be noted that the content of the active drug 210 being 0.5 µg/mm² to 6 µg/mm² refers to an average value of the content of the active drug 210 on the outer wall 110, the side walls 120 and the inner wall 130.

In an embodiment, the drug carrier is a degradable polymer. The active drug 210 is dispersed in the degradable polymer, and the release of the active drug 210 is controlled by the degradable polymer, which avoids the loss of most of the active drug 210 in the blood due to burst release.

In an embodiment, the degradable polymer is selected from at least one of a degradable polyester and a degradable anhydride. The degradable polyester is selected from at least one of polylactic acid, polyglycolic acid, polysuccinate, poly(beta-hydroxybutyrate), polycaprolactone, polyethylene glycol adipate, polyvalerate, polyhydroxy alkyl alcohol ester and poly(malate). Alternatively, the degradable polyester is a copolymer of at least two of the monomers forming the aforementioned degradable polyester type polymer. The degradable polyanhydride is selected from at least one of poly1,3-bis(p-carboxyphenoxy)propane-sebacic acid, polyerucic acid dimer-sebacic acid and polyfumaric acid-sebacic anhydride.

The degradation of the degradable polyester and the degradable polyanhydride generates a local slightly acidic environment around the substrate 100, which is beneficial to accelerating the later corrosion of the substrate 100.

In an embodiment, at least some regions of the portion of the drug coating 200 on the inner wall 130 are of a pore structure. Due to the pore structure, in the early stage of implantation, when the degradable polymer in the drug coating 200 degrades, few degradation products are produced, and the degradation products are able to diffuse relatively freely with body fluid, and do not aggregate locally to cause the local pH value to be too low, thereby avoiding the phenomenon of local significantly accelerated corrosion or degradation of the corrodible or degradable substrate 100.

In an embodiment, the pore structure of the portion of the drug coating 200 on the inner wall 130 has a porosity of 5% to 60%, which ensures that the degradation products of the degradable polymer diffuse out as quickly as possible and avoids too fast corrosion or degradation of the corrodible or degradable substrate 100.

In an embodiment, the thickness of the drug coating 200 is 2 microns to 20 microns. The thickness of the drug coating 200 refers to an average value of the thickness of the portion of the drug coating 200 on the outer wall 110, the thickness of the portion on the side walls 120 and the thickness of the portion on the inner wall 130.

The drug coating 200 covers the outer wall 110, the side walls 120 and the inner wall 130 of the substrate 100 at the same time, the thickness of the drug coating 200 is reasonably configured and the amount of the degradable polymer in the drug coating 200 is controlled, which ensures that the amount of the degradable polymer is sufficient, so that the degradation rate of the drug coating 200 matches the corrosion or degradation rate of the corrodible or degradable substrate 100, sufficient degradable polymer remains after the recovery of the lesion site is completed, and the degradation products of the remaining degradable polymer accelerate the corrosion or degradation of the substrate 100 to reduce the risk of long-term clinical use.

In an embodiment, the thickness of the portion of the drug coating 200 on the outer wall 110 and the thickness of the portion of the drug coating 200 on the side walls 120 are greater than the thickness of the portion of the drug coating 200 on the inner wall 130. After the drug eluting device is implanted in the body, the outer wall 110 of the substrate 100 is the side in direct contact with the wall of a blood vessel, and the active drug 210 on the outer wall 110 is controllably released and permeates into the intima of the blood vessel for better efficacy. Therefore, on the premise of avoiding excessive local thickness of the drug coating 200, the thickness of the portion of the drug coating 200 on the outer wall 110 being greater than the thickness of the portion of the drug coating 200 on the inner wall 130 is beneficial to increasing effective drug loading to meet the requirements of therapy.

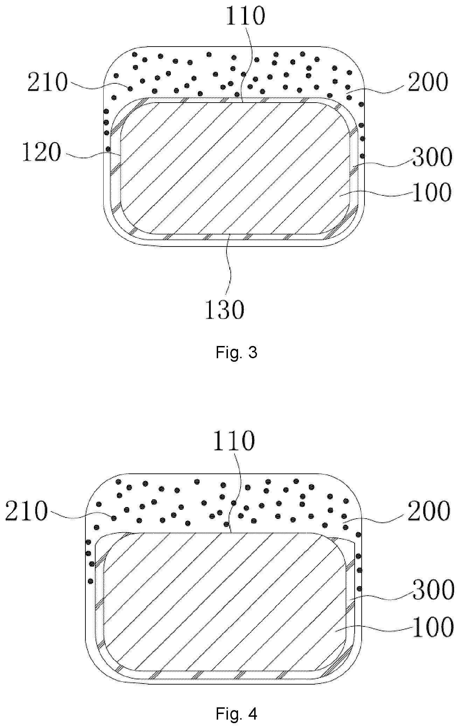

Referring to Fig. 3, in an embodiment, when the material of the substrate 100 is pure iron or iron alloy, the drug eluting device further includes a zinc-containing protective layer 300. The zinc-containing protective layer 300 is arranged on the substrate 100, and the zinc-containing protective layer 300 is covered by the drug coating 200. The zinc-containing protective layer 300 contains pure zinc, a zinc alloy and/or a zinc-containing compound. The zinc-containing protective layer 300 is provided on the substrate 100 to protect the substrate 100, thereby delaying the corrosion of the corrodible substrate 100 and maintaining the early radial support performance of the substrate 100.

The zinc-containing protective layer 300 covers at least the inner wall 130 of the substrate 100.

In an embodiment, the zinc-containing protective layer 300 covering at least the inner wall 130 of the substrate 100 means that the zinc-containing protective layer 300 completely covers the surface of the substrate 100, i.e., the zinc-containing protective layer completely covers the outer wall 110, the side walls 120 and the inner wall 130 of the substrate 100, as shown in Fig. 3.

In another embodiment, the zinc-containing protective layer 300 covering at least the inner wall 130 of the substrate 100 means that the zinc-containing protective layer 300 covers only the inner wall 130 of the substrate 100; or, the zinc-containing protective layer 300 completely covers the inner wall 130 of the substrate 100, the zinc-containing protective layer 300 only partially covers the side walls 120 of the substrate, and the zinc-containing protective layer 300 does not cover the outer wall 110 of the substrate 100 at all; or, the zinc-containing protective layer 300 completely covers the inner wall 130 and side walls 120 of the substrate 100, and the zinc-containing protective layer 300 only partially covers the outer wall 110 of the substrate 100; or, the zinc-containing protective layer 300 completely covers the inner wall 130 and the side walls 120 of the substrate 100, and the zinc-containing protective layer does not cover the outer wall 110 of the substrate 100 at all. The zinc-containing protective layer 300 covering only the inner wall 130 of the substrate 100 means that the zinc-containing protective layer 300 completely covers the inner wall 130 of the substrate 100, or the zinc-containing protective layer 300 only partially covers the inner wall 130 of the substrate 100, and the outer wall 110 and the side walls 120 of the substrate 100 are not covered by the zinc-containing protective layer 300 at all.

It should be noted that, for process control reasons, when the zinc-containing protective layer 300 is prepared on the substrate 100, some regions of the zinc-containing protective layer 300 on the substrate 100 may be discontinuous, e.g., some regions of the zinc-containing protective layer 300 on the substrate 100 are discontinuous due to the presence of missing plated regions. For example, the zinc-containing protective layer 300 covers only the inner wall 130 of the substrate 100, but some regions of the zinc-containing protective layer 300 are discontinuous. For another example, the zinc-containing protective layer 300 is located on the outer wall 110, the side walls 120 and the inner wall 130 of the substrate 100, and at least some regions of the portion of the zinc-containing protective layer 300 on the outer wall 110, the portion on the side walls 120 and the portion on the inner wall 130 are discontinuous. For still another example, the zinc-containing protective layer 300 is located on the inner wall 130 of the substrate 100, the zinc-containing protective layer 300 only partially covers the side walls 120 of the substrate 100, the zinc-containing protective layer 300 does not cover the outer wall 110 of the substrate 100 at all, and at least some regions of the portion of the zinc-containing protective layer 300 on the inner wall 130 and the portion on the side walls 120 are discontinuous. Alternatively, the zinc-containing protective layer 300 is located on the inner wall 130 and the side walls 120 of the substrate 100, the zinc-containing protective layer 300 only partially covers the outer wall 110 of the substrate 100 or the zinc-containing protective layer 300 does not cover the outer wall 110 of the substrate 100 at all, and at least some regions of the portion of the zinc-containing protective layer 300 on the inner wall 130, the portion on the side walls 120 and the portion on the outer wall 110 are discontinuous.

Regardless of how the zinc-containing protective layer 300 is distributed on the substrate 100, even if some regions of the zinc-containing protective layer 300 are discontinuous, the zinc-containing protective layer 300 can well protect the substrate 100.

The zinc-containing protective layer 300 covers at least the inner wall 130 of the substrate 100. After the drug eluting device is implanted into the blood vessel, the outer wall 110 of the substrate 100 abuts against the vessel wall, while the inner wall 130 of the substrate 100 is in contact with and flushed by the blood flow from time to time, and the zinc-containing protective layer 300 covering at least the inner wall 130 of the substrate 100 can well avoid premature corrosion of the corrodible substrate 100.

In an embodiment, the zinc-containing protective layer 300 has a coverage of 20% to 100% on the substrate 100, so that the zinc-containing protective layer 300 can protect the corrodible substrate 100 at an early stage and avoid premature corrosion of the corrodible substrate 100 without affecting the corrosion rate of the substrate 100 at a later stage. If the inner wall 130 of the substrate 100 does not contain zinc, corrosion may occur early, causing thrombogenicity, thus requiring the zinc-containing protective layer 300 to cover at least the inner wall 130 of the substrate 100. In addition, because the outer wall 110 of the substrate 100 is not in contact with blood, in order to increase the corrosion rate of the whole substrate 100, the outer wall 110 of the substrate 100 may not be covered by the zinc-containing protective layer 300 or the outer wall 110 may be only partially covered by the zinc-containing protective layer 300.

In an embodiment, as shown in Fig. 4, at least part of the outer wall 110 of the substrate 100 is not covered by the zinc-containing protective layer 300. In order to increase the effective drug loading of the substrate 100, the portion of the drug coating 200 on the outer wall 110 of the substrate 100 is thicker. On the premise that the zinc-containing protective layer 300 can well protect the substrate 100 to avoid premature corrosion of the substrate 100, at least part of the outer wall 110 of the substrate 100 is not covered by the zinc-containing protective layer 300, which is beneficial to accelerating the corrosion of the substrate 100 from the outer wall 110 at the later stage and shortening the corrosion period.

Through the complete, continuous, and asymmetric loaded drug coating 200, the drug eluting device avoids the release of the excessive active drug 210 from the inner wall 130 of the substrate 100 to the blood to cause toxic and side effects, which facilitates the climbing of endothelial cells on the inner wall 130 of the substrate 100 and effectively avoids falling of the drug coating 200 during expansion. In addition, in an embodiment, the substrate 100 is made of an iron-based alloy having a carbon content of not more than 2.11 wt.% or pure iron, the active drug 210 in the drug coating 200 has a content of 0.5 µg/mm² to 6 µg/mm², the drug coating 200 has a thickness of 2 to 20 microns, the pore structure of the portion of the drug coating 200 on the inner wall 130 has a porosity of 5% to 60%, so that the release of the active drug 210 matches the corrosion of the corrodible substrate 100, the release period of the active drug 210 coincides with the recovery period of the implanted site, and the corrodible substrate 100 retains sufficient radial support force during the recovery period; and after the recovery is completed, the active drug 210 is completely released, and the degradable polymer is not completely degraded, and can be continuously degraded to maintain a local slightly acidic environment around the substrate 100 to rapidly corrode the corrodible substrate 100, thereby reducing long-term clinical risk.

In an embodiment, the substrate 100 of the drug eluting device is made of an iron-based alloy having a carbon content of not more than 2.11 wt.% or pure iron, the active drug 210 in the drug coating 200 has a content of 0.5 µg/mm² to 6 µg/mm², the drug coating 200 has a thickness of 2 microns to 20 microns, and the substrate 100 is provided with the zinc-containing protective layer 300 covering at least the inner wall 100 of the substrate 100, which can delay the initiation of corrosion of the substrate 100, so that the release of the active drug 210 matches the corrosion of the corrodible substrate 100, the release period of the active drug 210 coincides with the recovery period of the implanted site, and the corrodible substrate 100 retains sufficient radial support force during the recovery period; and after the recovery is completed, the corrodible substrate 100 rapidly corrodes, thereby reducing long-term clinical risk.

Further, the substrate 100 of the drug eluting device is made of an iron-based alloy having a carbon content of not more than 2.11 wt.% or pure iron, the active drug 210 in the drug coating 200 has a content of 0.5 µg/mm² to 6 µg/mm², the drug coating 200 has a thickness of 2 microns to 20 microns, the pore structure of the portion of the drug coating 200 on the inner wall 130 has a porosity of 5% to 60%, and the substrate 100 is provided with the zinc-containing protective layer 300 covering at least the inner wall 100 of the substrate 100, which can further ensure that the release of the active drug 210 matches the corrosion of the corrodible substrate 100, so that the release period of the active drug 210 coincides with the recovery period of the implanted site, and the corrodible substrate 100 retains sufficient radial support force during the recovery period; and after the recovery is completed, the corrodible substrate 100 rapidly corrodes, thereby reducing long-term clinical risk.

The above-mentioned drug eluting device may be a device applied to various sites. For example, the drug eluting device is a cardiovascular stent, a cerebrovascular stent, a peripheral vascular stent, a biliary stent, an esophageal stent, an airway stent or an orthopedic implant.

A preparation method of a drug eluting device according to an embodiment includes the following steps:
Step 110A: a substrate is provided, the substrate having an outer wall, side walls and an inner wall.

The substrate is of a hollowed lumen structure.

Step 120: a solution containing an active drug and a drug carrier is applied to the substrate to form a drug coating, the active drug is dispersed in the drug carrier, and the drug coating covers the outer wall, the side walls, and the inner wall of the substrate.

The active drug and the drug carrier are dissolved in a solvent to prepare the solution containing the active drug and the drug carrier. In an embodiment, when the drug carrier is a degradable polymer, the active drug and the degradable polymer are dissolved in a solvent, fully dissolved, and uniformly stirred to obtain the solution containing the active drug and the drug carrier. Or, the active drug and the degradable polymer are respectively dissolved in different solvents to prepare a solution containing the active drug and a solution containing the drug carrier, and then the solution containing the active drug is mixed with the solution containing the drug carrier to obtain a mixed solution of the active drug and the drug carrier.

In an embodiment, the solution containing the active drug and the drug carrier is applied to the substrate by spraying, leaching, dipping, or rolling to form the drug coating. The drug coating coats the outer wall, the side walls, and the inner wall of the substrate. In this embodiment, the drug coating is of a single-layer structure, the active drug is dispersed in the drug carrier, and the active drug is non-uniformly dispersed in the drug carrier.

It should be noted that, in the solution containing the active drug and the drug carrier, the concentration of the active drug and the concentration of the drug carrier are selected as required without specific limitations.

It should also be noted that the above step may be repeated multiple times to obtain the desired thickness of the drug coating.

Step 130: a solvent is brought into contact with a portion of the drug coating on the inner wall such that the active drug at the portion of the drug coating on the inner wall is partially or completely dissolved in the solvent and the drug carrier is insoluble in the solvent, and the solvent is removed from the substrate.

In an embodiment, an adsorbent material adsorbing the solvent is brought into contact with the inner wall of the substrate such that the active drug at the portion of the drug coating on the inner wall is partially or completely dissolved in the solvent and the drug carrier is insoluble in the solvent, then the adsorbent material is removed, and the solvent is thus removed from the substrate.

It should be noted that the active drug at the portion of the drug coating on the inner wall is partially dissolved or completely dissolved in the solvent, and the drug carrier is insoluble in the solvent, which does not necessarily require that the drug carrier is completely insoluble in the solvent, but which refers to that the differences in solubility of the active drug and the drug carrier are great with respect to the same solvent, or that the dissolution of the drug carrier in the solvent is slow, and within the time of contact between the solvent and the portion of the drug coating on the inner wall, the active drug can be quickly dissolved in the solvent, while the drug carrier is dissolved in the solvent in a negligible amount without disrupting the continuity of the portion of the drug coating on the inner wall.

In an embodiment, the solvent is methanol, ethanol, propanol, isopropanol, acetonitrile or ethyl acetate.

In an embodiment, the adsorbent material is a nylon material, polypropylene, a polytetrafluoroethylene material, a polyvinyl chloride material, a rubber-like material, or a fiber-like material, specifically a non-woven fabric adsorbent cotton made of a polypropylene material.

The adsorbent material not only has adsorbability and can adsorb a solvent, but also does not contaminate the substrate in the process of contacting the substrate. It could be appreciated that in addition to the materials listed above, any adsorbent material capable of adsorbing a solvent without contaminating the substrate can be used.

The adsorbent material adsorbing the solvent is brought into contact with the inner wall of the substrate, such that the active drug at the portion of the drug coating on the inner wall is partially or completely dissolved in the solvent, the drug carrier is insoluble in the solvent, the active drug at the portion of the drug coating on the inner wall is at least partially removed, and at least some regions of the portion of the drug coating on the inner wall become pore structures with suitable porosity. In order to ensure that a certain amount of active drug can be removed and ensure that the pore structure of the portion of the drug coating on the inner wall has a porosity of 5% to 60%, in an embodiment, the time for contact between the adsorbent material adsorbing the solvent and the inner wall of the substrate is 10 s to 20 min.

Specifically, in an embodiment, the step of bringing the adsorbent material adsorbing the solvent into contact with the inner wall of the substrate such that the active drug at the portion of the drug coating on the inner wall is partially or completely dissolved in the solvent, and then removing the adsorbent material includes: a core shaft is provided, the outer surface of the core shaft is wrapped with the adsorbent material, the core shaft wrapped with the adsorbent material is put into the interior of the substrate, the adsorbent material is caused to adsorb the solvent, the adsorbent material adsorbing the solvent is brought into contact with the inner wall of the substrate such that the active drug at the portion of the drug coating on the inner wall is partially or completely dissolved in the solvent, and then the adsorbent material and the core shaft are removed from the substrate.

Alternatively, in another embodiment, a core shaft is provided, the outer surface of the core shaft is wrapped with the adsorbent material, the adsorbent material is caused to adsorb the solvent, then the core shaft wrapped with the adsorbent material is put into the interior of the substrate, the adsorbent material is brought into contact with the inner wall of the substrate such that the active drug at the portion of the drug coating on the inner wall is partially or completely dissolved in the solvent, and then the adsorbent material and the core shaft are removed from the substrate.

The core shaft is cylindrical to fit the substrate of the lumen structure. In the embodiment in which the outer surface of the core shaft is wrapped with the adsorbent material, the material of the core shaft is not limited as long as the adsorbent material can be supported.

In another embodiment, the cylindrical core shaft may be formed directly from the adsorbent material. In this way, the surface of the core shaft does not need to be additionally wrapped with the adsorbent material, the core shaft is directly used to adsorb the solvent that can dissolve the active drug but hardly dissolves or does not dissolve the drug carrier, then the core shaft adsorbing the solvent is put into the substrate and brought into contact with the inner wall of the substrate such that the active drug at the portion of the drug coating on the inner wall is dissolved in the solvent, and then the core shaft is removed from the substrate. Alternatively, the core shaft is put into the substrate, the core shaft is caused to adsorb the solvent and brought into contact with the inner wall of the substrate such that the active drug at the portion of the drug coating on the inner wall is dissolved in the solvent, and then the core shaft is removed from the substrate.

It should be noted that whether the core shaft is wrapped with the adsorbent material or the core shaft is formed from the adsorbent material, the core shaft is rotated during the operation of removing the active drug from the inner wall of the substrate or reducing the active drug on the inner wall of the substrate. Alternatively, in another embodiment, the core shaft remains stationary while the substrate is in a rotated state.

In another embodiment, the inner wall of the substrate is directly wiped with the absorbent material absorbing the solvent such that the active drug at the portion of the drug coating on the inner wall is partially or completely dissolved in the solvent and the drug carrier is insoluble in the solvent, and then the adsorbent material is removed.

It should also be noted that regardless of the mode in which the active drug is removed from the inner wall of the substrate or the active drug on the inner wall of the substrate is reduced, the corresponding operation can be repeated multiple times to reduce the active drug on the inner wall of the substrate as much as possible or to completely remove the active drug from the inner wall of the substrate while ensuring that the portion of the drug coating on the inner wall is a continuous coating. It can be estimated that during the process of contact between the material adsorbing the solvent and the inner wall of the substrate, part of the side walls of the substrate may be contacted, so that the drug at a portion, close to the inner wall, of the side walls of the substrate is also reduced.

According to the preparation method of the drug eluting device, the continuous drug coating covering the outer wall, the side walls and the inner wall of the substrate is firstly prepared, and then the active drug at the portion of the drug coating on the inner wall is at least partially removed. The content of the active drug in the drug eluting device obtained after step 130 is reduced by up to 50% over the content of the active drug in the semi-finished drug eluting device obtained after step 120.

It could be appreciated that after the active drug at the portion of the drug coating on the inner wall is removed or reduced, a drying step is further included to volatilize the solvent of the drug coating as quickly as possible. In an embodiment, the drying may be performed by means of a drying agent, ventilation, vacuumizing, etc. In other embodiments, natural air drying is also possible.

It could also be appreciated that when multiple layers of drug coatings are required to be formed on the substrate, the multiple layers of drug coatings can be sequentially prepared by the same method described above.

According to the preparation method of the drug eluting device, the complete and continuous drug coating that completely coats the outer wall, the inner wall and the side walls of the substrate is prepared by means of the obvious difference in solubilities of the active drug and the drug carrier in the same solvent, and the content of the active drug in the drug coating both on the outer wall and the side walls is greater than the content on the inner wall; or, the active drug is distributed only on the outer wall and the side walls, and the inner wall does not contain the active drug. Therefore, the clinical use safety is improved. In addition, the preparation method of the drug eluting device is simple in process and low in equipment requirements.

In other embodiments, before the drug coating is prepared, a zinc-containing protective layer attached to the substrate is further prepared. The zinc-containing protective layer may be prepared by means of electroplating, spraying, dipping, brushing, electrostatic spinning, etc.

The drug eluting device and the preparation method thereof are further illustrated by the following specific embodiments.

Test methods used by the following embodiments are as follows:
1. Test on endothelialization rate: the drug eluting device is implanted into an iliac artery of a rabbit, the blood vessel where the drug eluting device is located is taken out after a certain time, soaked into glutaraldehyde (for example, 6 h), dried, then cut axially, and subjected to metal spraying, and the endothelial coverage of the drug eluting device is measured and observed by using an SEM (Scanning Electron Microscope), when the coverage reaches more than 95% of the area, the drug eluting device completes endothelialization.
2. Evaluation on completeness of the drug coating: peeling test, that is, after the pushing of the drug eluting device system is simulated in a PBS simulation solution, the drug eluting device is expanded to a blasting pressure, and the appearance of the drug coating is observed with a 20-50 times microscope, the drug coating is not obviously peeled in a large area from the substrate of the drug eluting device, the coating is peeled off, and the substrate is not obviously exposed on the outer wall of the drug eluting device.
3. Test on corrosion rate of the absorbable drug eluting device in vivo: the corrosion rate of the drug eluting device in vivo can be characterized by the mass loss of the drug eluting device. For example, after the absorbable drug eluting device is implanted into a rabbit for a certain time, the drug eluting device and surrounding tissues are taken out, the drug eluting device is ultrasonically cleaned with a tartaric acid solution (e.g. 3% by mass) and dried, and then the mass MI of the drug eluting device is measured, if the mass of the original drug eluting device as a bare stent is M₀, the mass loss of the drug eluting device is (M₀-M₁)/M₀. The initiation of corrosion of the drug eluting device indicates that the mass loss of the device is 5-10%.
4. Characterization on the asymmetric effect of content of the active drug of the drug eluting device: the asymmetric effect can be characterized by Raman spectroscopy, and the specific characterization method is: Raman spectrum analysis is performed on the portions of the drug coating on the outer wall and the inner wall by using a Raman spectrometer of Thermo Fisher Scientific under the wavelength condition of 532 nm; when the outer wall shows a characteristic peak of the active drug and the inner wall does not, the outer wall has the active drug and the inner wall does not have the active drug; and when the intensity of the characteristic peak of the active drug on the outer wall is obviously higher than that on the inner wall, the content of the active drug on the inner wall is lower than that on the outer wall.
5. Test on drug loading of the drug eluting device before and after the active drug on the inner wall is removed: the drug eluting device is immersed in acetonitrile of a certain volume while ensuring that the acetonitrile fully immerses the drug eluting device, and ultrasonically treated for a certain time (e.g. 30 min), and the drug content in the solution is tested by means of Agilent 1260 liquid chromatography, the equipment parameter is an ultraviolet wavelength of 278 nm, the mobile phase includes acetonitrile and water in a ratio of 65:35, and the pump flow rate is 1 mL/min. The drug contents of the representative sample of the current batch of drug eluting device before and after the drug on the inner wall is removed are respectively measured as M₀ µg and M₁ µg, then the drug content of the drug eluting device is reduced by (M₀-M₁)*100%/M₀, and the content of the active drug on the stent can also be expressed by the drug content per unit area: Mi/S, where S is the surface area mm² of a stent rod.
6. Test on thickness of the coating: metal spraying, resin embedding and grinding treatment are performed on the prepared drug eluting device, the dimension of each cross section in each direction is observed and measured under the scanning electron microscope, typical cross sections are taken, the thickness of the coating at a middle position in each direction is respectively taken for the drug coating on each cross section of the inner wall, the outer wall and the side walls, and the average value of the thicknesses is used as the thickness of the drug coating.
7. Porosity of the pore structure of the portion of the drug coating on the inner wall: a percentage of the volume occupied by pores of the portion of the drug coating on the inner wall to the total volume of the portion of the drug coating on the inner wall. Since the amount of the drug coating is small and the precision of the conventional porosity test method is low, an SEM method is used for rough evaluation. The specific operation method is: axial brittle fracture and metal spraying are performed on the drug eluting device, the coating on the inner wall of each stent rod is observed, at least three axial cross sections are selected, at least five 2*2 µm areas are selected on each cross section for photographing, then the ratio of the pore area to the area of the selected area is measured and calculated uniformly by statistical software, and an average value of the 15 ratios is used as the porosity.
8. Evaluation on coverage of the zinc-containing protective layer on the surface of the substrate of the drug eluting device: the evaluation is performed by means of microscopic.

Taking drug eluting stents with specifications of 30008 (expanded outer diameter of 3 mm and length of 8 mm under the nominal pressure), 40008 (expanded outer diameter of 4 mm and length of 8 mm under the nominal pressure) and 60008 (expanded outer diameter of 6 mm and length of 8 mm under the nominal pressure) as examples, the drug eluting device and the preparation method thereof are further illustrated.

### Embodiment 1

A mixed solution of PDLLA and sirolimus was prepared, the concentrations of the PDLLA and the sirolimus were 6 mg/mL, and the solvent was acetone. The mixed solution was coated on an outer wall, side walls and an inner wall of a substrate of the stent of 30008 specification, and a drug coating that covered the outer wall, the side walls and the inner wall of the substrate was formed after drying. The material of the substrate was pure iron.

The sirolimus content of this batch of stents was measured to be 2.0 µg/mm². A core shaft made of a porous nylon material penetrated through the inner wall of the substrate of the stent, the core shaft fully adsorbed ethanol, then the core shaft expanded to contact a portion of the drug coating on the inner wall for 2 min, the core shaft was taken out, and the stent was fully dried.

This batch of stents were taken, and the final sirolimus content of this batch of stents was measured to be 1.5 µg/mm², which was 25% less than that of the stents before the solvent was adsorbed. The thickness of the drug coating was measured to be 5 µm. The Raman spectrometer test showed that the drug content of the portion of the drug coating on the inner wall of the substrate was lower than that of a portion on the outer wall. The porosity of the portion of the drug coating on the inner wall of the substrate was measured to be 35%. The peeling test results showed that the coating was complete after the stent was pushed by simulation and expanded to a bursting pressure. A plurality of stents prepared in this batch were respectively implanted into iliac arteries of a plurality of rabbits, samples were taken in different time periods, and the tests showed that the stents were completely endothelialized after being implanted for 2 months; the stents had mass losses of 25% after being implanted for 3 months; and the stents had mass losses of 50% after being implanted for 1 year.

### Embodiment 2

A mixed solution of PDLLA and sirolimus was prepared, wherein the concentrations of the PDLLA and the sirolimus were respectively 4 mg/mL and 6 mg/mL, and the solvent was acetone. The mixed solution was coated on an outer wall, side walls and an inner wall of a substrate of the stent of 30008 specification, and a drug coating that covered the outer wall, the side walls and the inner wall of the substrate was formed after drying. The material of the substrate was pure iron.

The sirolimus content of this batch of stents was measured to be 2.5 µg/mm², the surface of a cylindrical core shaft was wrapped with a porous nylon material, the core shaft penetrated through the inner wall of the substrate of the stent, the porous nylon material fully adsorbed ethanol, then the core shaft expanded to contact a portion of the drug coating on the inner wall for 10 min, the core shaft was taken out, and the stent was fully dried.

This batch of stents were taken, and the final sirolimus content of this batch of stents was measured to be 1.5 µg/mm², which was 40% less than that of the stents before the solvent was absorbed. The thickness of the drug coating was measured to be 5 µm. The Raman spectrometer test showed that the portion of the drug coating on the inner wall of the substrate contained no drug. The porosity of the portion of the drug coating on the inner wall of the substrate was measured to be 55%. The peeling test results showed that the coating was complete after the stent was pushed by simulation and expanded to a bursting pressure. A plurality of stents prepared in this batch were respectively implanted into iliac arteries of a plurality of rabbits, samples were taken in different time periods, and the tests showed that the stents were completely endothelialized after being implanted for 1.5 months; the stents had mass losses of 20% after being implanted for 3 months; and the stents had mass losses of 50% after being implanted for 1 year.

### Embodiment 3

A mixed solution of PDLLA and sirolimus was prepared, wherein the concentrations of the PDLLA and the sirolimus were respectively 3 mg/mL and 6 mg/mL, and the solvent was acetone. The mixed solution was coated on an outer wall, side walls and an inner wall of a substrate of the stent of 30008 specification, and a drug coating that covered the outer wall, the side walls and the inner wall of the substrate was formed after drying. The material of the substrate was pure iron. The substrate was provided with a zinc-containing protective layer, the zinc-containing protective layer covered part of the outer wall, all of the side walls and the inner wall of the substrate, the coverage of the zinc-containing protective layer was 90%, and the material of the zinc-containing protective layer was pure zinc. The drug coating covered the surface of the zinc-containing protective layer.

The sirolimus content of this batch of stents was measured to be 2.0 µg/mm². The surface of a stainless-steel core shaft was wrapped with a porous polystyrene material, the core shaft penetrated through the inner wall of the substrate of the stent, the porous polystyrene material fully adsorbed ethanol, then the porous polystyrene material expanded to contact a portion of the drug coating on the inner wall for 2 min, the core shaft was taken out, and the stent was fully dried.

This batch of stents were taken, and the final sirolimus content of this batch of stents was measured to be 1.5 µg/mm², which was 25% less than that of the stents before the solvent was absorbed. The thickness of the drug coating was measured to be 4 µm. The Raman spectrometer test showed that the drug content of the portion of the drug coating on the inner wall of the substrate was lower than that on the outer wall. The porosity of the portion of the drug coating on the inner wall of the substrate was measured to be 45%. The peeling test results showed that the coating was complete after the stent was pushed by simulation and expanded to a bursting pressure. A plurality of stents prepared in this batch were respectively implanted into iliac arteries of a plurality of rabbits, samples were taken in different time periods, and the tests showed that the stents were completely endothelialized after being implanted for 2 months; the corrosion of the stents was just initiated after the stents were implanted for 3 months; and the stents had mass losses of 40% after being implanted for 1 year.

### Embodiment 4

A mixed solution of 4 mg/mL PLLA and 5 mg/mL sirolimus was prepared by using tetrahydrofuran as a solvent, the mixed solution was coated on an outer wall, side walls and an inner wall of a substrate of the stent of 30008 specification, and a drug coating that covered the substrate was formed after drying. The material of the substrate was an iron-based alloy with a carbon content of not more than 2.11 wt.%, the substrate was provided with a zinc-containing protective layer, the zinc-containing protective layer only covered all surfaces of the side walls and the inner wall of the substrate, the outer wall was not covered with zinc, the coverage of the zinc-containing protective layer on the substrate was 70%, and the material of the zinc-containing protective layer was pure zinc. The drug coating covered the surface of the zinc-containing protective layer.

The sirolimus content of this batch of stents was measured to be 5 µg/mm². The surface of a stainless-steel core shaft was wrapped with a porous polystyrene material, the core shaft penetrated through the inner wall of the substrate of the stent, the porous polystyrene material fully adsorbed methanol, then the porous polystyrene material expanded to contact a portion of the drug coating on the inner wall for 10 s, the core shaft was taken out, and the stent was fully dried.

This batch of stents were taken, and the final sirolimus content of this batch of stents was measured to be 4.5 µg/mm², which was 10% less than that of the stents before the solvent was adsorbed. The thickness of the drug coating was measured to be 8 µm. The Raman spectrometer test showed that the drug content of the portion of the drug coating on the inner wall of the substrate was lower than that of a portion on the outer wall. The porosity of the portion of the drug coating on the inner wall of the substrate was measured to be 15%. The peeling test results showed that the coating was complete after the stent was pushed by simulation and expanded to a bursting pressure. A plurality of stents prepared in this batch were respectively implanted into iliac arteries of a plurality of rabbits, samples were taken in different time periods, and the tests showed that the stents were completely endothelialized after being implanted for 2.5 months; the corrosion of the stents was just initiated after the stents were implanted for 3 months; and the stents had mass losses of 50% after being implanted for 1 year.

### Embodiment 5

A mixed solution of 6 mg/mL PLGA and 10 mg/mL sirolimus was prepared by using ethyl acetate as a solvent, the mixed solution was uniformly sprayed to an outer wall, side walls and an inner wall of a substrate of the stent of 40008 specification, and a drug coating that covered the substrate was formed after drying. The material of the substrate was an iron-based alloy with a carbon content of not more than 2.11 wt.%, the substrate was provided with a zinc-containing protective layer, the zinc-containing protective layer covered the outer wall, the side walls and the inner wall of the substrate, the coverage of the zinc-containing protective layer on the substrate was 100%, the drug coating covered the surface of the zinc-containing protective layer, and the drug coating also covered the outer wall, the side walls and the inner wall of the substrate. The material of the zinc-containing protective layer was pure zinc.

The sirolimus content of this batch of stents was measured to be 10 µg/mm². A sponge stick dipped with acetonitrile was rotated forward on the inner wall of the substrate to uniformly wipe the inner wall of the substrate repeatedly, wherein the time for contact between the sponge stick dipped with acetonitrile and the inner wall of the stent was 20 min, so that the sponge stick dipped with acetonitrile adsorbed sirolimus at a portion of the drug coating on the inner wall; and the stent was fully dried.

This batch of stents were taken, and the final sirolimus content of this batch of stents was measured to be 6 µg/mm², which was 40% less than that of the stents before the solvent was adsorbed. The thickness of the drug coating was measured to be 15 µm. The Raman spectrometer test showed that the portion of the drug coating on the inner wall of the substrate contained no drug. The porosity of the portion of the drug coating on the inner wall of the substrate was measured to be 60%. The peeling test results showed that the coating was complete after the stent was pushed by simulation and expanded to a bursting pressure. A plurality of stents prepared in this batch were respectively implanted into iliac arteries of a plurality of rabbits, samples were taken in different time periods, and the tests showed that the stents were completely endothelialized after being implanted for 2 months; the corrosion of the stents was not initiated after the stents were implanted for 3 months; and the stents had mass losses of 65% after being implanted for 1 year.

### Embodiment 6

A mixed solution of 6 mg/mL crystalline PLLA and 0.5 mg/mL paclitaxel was prepared by using trichloromethane as a solvent, the mixed solution was uniformly sprayed to an outer wall, side walls and an inner wall of a substrate of the stent having a diameter of 30008 specification, and a drug coating that covered the outer wall, the side walls and the inner wall of the substrate was formed after drying. The material of the substrate was pure iron. The substrate was provided with a zinc-containing protective layer, the zinc-containing protective layer covered all of the inner wall and part of the side walls of the substrate, the coverage of the zinc-containing protective layer on the substrate was 60%, and the material of the zinc-containing protective layer was pure zinc. The drug coating covered the surface of the zinc-containing protective layer.

The paclitaxel content of this batch of stents was measured to be 1 µg/mm². A dust-free fabric dipped with ethyl acetate was brought into contact with the inner wall of the substrate for 8 min, so that ethyl acetate adsorbed the drug at a portion of the drug coating on the inner wall; and the stent was fully dried.

This batch of stents were taken, and the final paclitaxel content of this batch of stents was measured to be 0.95 µg/mm², which was 5% less than that of the stents before the solvent was adsorbed. The thickness of the drug coating was measured to be 10 µm. The Raman spectrometer test showed that the drug content of the portion of the drug coating on the inner wall of the substrate was lower than that of a portion on the outer wall. The porosity of the portion of the drug coating on the inner wall of the substrate was measured to be 5%. The peeling test results showed that the coating was complete after the stent was pushed by simulation and expanded to a bursting pressure. A plurality of stents prepared in this batch were respectively implanted into iliac arteries of a plurality of rabbits, samples were taken in different time periods, and the tests showed that the stents were completely endothelialized after being implanted for 2 months; the corrosion of the stents was just initiated after the stents were implanted for 3 months; and the stents had mass losses of 65% after being implanted for 1 year.

### Embodiment 7

A mixed solution of 4 mg/mL PLGA and 4 mg/mL sirolimus derivative was prepared by using ethyl acetate as a solvent, the mixed solution was uniformly sprayed to an outer wall, side walls and an inner wall of a substrate of the stent having a diameter of 30008 specification, and a drug coating that covered the outer wall, the side walls and the inner wall of the substrate was formed after drying. The material of the substrate was pure iron.

The sirolimus derivative content of this batch of stents was measured to be 0.8 µg/mm². A polypropylene non-woven fabric dipped with isopropanol was brought into contact with the inner wall of the substrate for 5 min, so that isopropanol adsorbed the drug at a portion of the drug coating on the inner wall; and the stent was fully dried.

This batch of stents were taken, and the final sirolimus derivative content of this batch of stents was measured to be 0.5 µg/mm², which was 38% less than that of the stents before the solvent was adsorbed. The thickness of the drug coating was measured to be 2 µm. The Raman spectrometer test showed that the portion of the drug coating on the inner wall of the substrate contained no drug. The porosity of the portion of the drug coating on the inner wall of the substrate was measured to be 50%. The peeling test results showed that the coating was complete after the stent was pushed by simulation and expanded to a bursting pressure. A plurality of stents prepared in this batch were respectively implanted into iliac arteries of a plurality of rabbits, samples were taken in different time periods, and the tests showed that the stents were completely endothelialized after being implanted for 1 months; the stents had mass losses of 15% after being implanted for 3 months; and the stents had mass losses of 40% after being implanted for 1 year.

### Embodiment 8

A 5 mg/mL crystalline PLLA solution and a mixed solution of 5 mg/mL PLLA and 1 mg/mL sirolimus were prepared by using trichloromethane as a solvent, the PLLA solution was first uniformly sprayed to an outer wall, side walls and an inner wall of a substrate of the stent having a diameter of 60008 specification, and a base coating that covered the outer wall, the side walls and the inner wall of the substrate was formed after drying. Then, the mixed solution of PLLA and sirolimus was uniformly sprayed to the surface of the stent to form a drug-loaded coating, wherein the drug-loaded coating covered the base coating, and the material of the substrate was pure iron. The substrate was provided with a zinc-containing protective layer, the zinc layer covered the surfaces of the outer wall, the side walls, and the inner wall of the substrate at the same time, the coverage of the zinc-containing protective layer on the substrate was 100%, and the material of the zinc-containing protective layer was pure zinc. The drug coating covered the surface of the zinc-containing protective layer.

The sirolimus content of this batch of stents was measured to be 4 µg/mm². A polypropylene non-woven fabric dipped with acetonitrile was brought into contact with the inner wall of the substrate for 15 min, so that acetonitrile adsorbed the drug at a portion of the drug coating on the inner wall; and the stent was fully dried.

This batch of stents were taken, and the final sirolimus content of this batch of stents was measured to be 3.2 µg/mm², which was 20% less than that of the stents before the solvent was adsorbed. The thickness of the drug coating was measured to be 20 µm. The Raman spectrometer test showed that the drug content of the portion of the drug coating on the inner wall of the substrate was lower than that of a portion on the outer wall. The porosity of the portion of the drug coating on the inner wall of the substrate was measured to be 10%. The peeling test results showed that the coating was complete after the stent was pushed by simulation and expanded to a bursting pressure. A plurality of stents prepared in this batch were respectively implanted into iliac arteries of a plurality of rabbits, samples were taken in different time periods, and the tests showed that the stents were completely endothelialized after being implanted for 2.5 months; the corrosion of the stents was just initiated after the stents were implanted for 3 months; and the stents had mass losses of 70% after being implanted for 1 year.

### Embodiment 9

A mixed solution of 3 mg/mL PLGA and 5 mg/mL sirolimus was prepared by using ethyl acetate as a solvent, the mixed solution was uniformly sprayed to an outer wall, side walls and an inner wall of a substrate of the stent of 30008 specification, and a drug coating that covered the substrate was formed after drying. The material of the substrate was an iron-based alloy with a carbon content of not more than 2.11 wt.%, the substrate was provided with a zinc-containing protective layer, the zinc-containing protective layer only covered most of the surface of the inner wall of the substrate, the coverage of the zinc-containing protective layer on the substrate was 20%, and the material of the zinc-containing protective layer was pure zinc. The drug coating covered the surface of the zinc-containing protective layer.

The sirolimus content of this batch of stents was measured to be 4 µg/mm². A sponge stick dipped with propanol was rotated forward on the inner wall of the substrate to uniformly wipe the inner wall of the substrate repeatedly, wherein the time for contact between the sponge stick dipped with propanol and the inner wall of the stent was 15 min, so that the sponge stick dipped with propanol adsorbed the sirolimus at a portion of the drug coating on the inner wall; and the stent was fully dried.

This batch of stents were taken, and the final sirolimus content of this batch of stents was measured to be 2 µg/mm², which was 50% less than that of the stents before the solvent was adsorbed. The thickness of the drug coating was measured to be 7 µm. The Raman spectrometer test showed that the drug content of the portion of the drug coating on the inner wall of the substrate was lower than that of a portion on the outer wall. The porosity of the portion of the drug coating on the inner wall of the substrate was measured to be 60%. The peeling test results showed that the coating was complete after the stent was pushed by simulation and expanded to a bursting pressure. A plurality of stents prepared in this batch were respectively implanted into iliac arteries of a plurality of rabbits, samples were taken in different time periods, and the tests showed that the stents were completely endothelialized after being implanted for 1 months; the corrosion of the stents was just initiated after the stents were implanted for 3 months; and the stents had mass losses of 50% after being implanted for 1 year.

### Comparative Example 1

A mixed solution of 6 mg/mL PDLLA and 6 mg/mL sirolimus was prepared by using acetone as a solvent, the mixed solution was coated on an outer wall and side walls of a substrate of the stent of 30008 specification, a drug coating that covered the outer wall and the side walls of the substrate was formed after drying, and an inner wall of the substrate was not covered by the drug coating. The material of the substrate was pure iron.

This batch of stents were taken, the sirolimus on this batch of stents was measured to be 1.5 µg/mm², and the thickness of the coating was measured to be 5 µm. The Raman spectrometer test showed that the outer wall and the side walls of the substrate contained the drug and the inner wall contained no drug; and the peeling test showed that the coating was obviously damaged and tilted after the stent was pushed by simulation and expanded to a blasting pressure, and the phenomenon that the coating was peeled from the substrate in a large area occurred. A plurality of stents prepared in this batch were respectively implanted into iliac arteries of a plurality of rabbits, samples were taken in different time periods, and the tests showed that the stents were completely endothelialized after being implanted for 1.5 months; the stents had mass losses of 15% after being implanted for 3 months; and the stents had mass losses of 40% after being implanted for 1 year.

### Comparative Example 2

A mixed solution of 4 mg/mL PDLLA and 6 mg/mL sirolimus was prepared by using acetone as a solvent, the mixed solution was coated on an outer wall, side walls and an inner wall of a substrate of the stent of 30008 specification, and a drug coating that covered the outer wall, the side walls and the inner wall of the substrate was formed after drying. The material of the substrate was pure iron.

This batch of stents were taken, the sirolimus on this batch of stents was measured to be 2.5 µg/mm², and the thickness of the coating was measured to be 5 µm. The Raman spectrometer test showed that the drug content of a portion of the drug coating on the inner wall of the substrate was not obviously different from that of a portion on the outer wall. The peeling test results showed that the coating was complete after the stent was pushed by simulation and expanded to a bursting pressure. A plurality of stents prepared in this batch were respectively implanted into iliac arteries of a plurality of rabbits, samples were taken in different time periods, and the tests showed that the stents were completely endothelialized and had mass losses of 28% after being implanted for 3 months; and the stents had mass losses of 50% after being implanted for 1 year.

The technical features of the above-described embodiments can be combined arbitrarily. For the purpose of simplicity in description, all the possible combinations of the technical features in the above embodiments are not described. However, as long as the combinations of these technical features do not have contradictions, they shall fall within the scope of the specification.

The foregoing embodiments only describe several implementations of the present disclosure, and their descriptions are specific and detailed, but cannot therefore be understood as limitations to the patent scope of the present disclosure. It should be noted that a person of ordinary skill in the art could also make several alterations and improvements without departing from the spirit of the present disclosure and these would all fall within the scope of protection of the present disclosure. Therefore, the protection scope of the present disclosure shall be in accordance with the appended claims.

## Claims

1. A drug eluting device, comprising a substrate and a drug coating arranged on the substrate, the substrate having an outer wall, side walls and an inner wall,
wherein the drug coating contains active drug and a drug carrier, the active drug is dispersed in the drug carrier, the drug coating covers the outer wall, the side walls, and the inner wall of the substrate, and both of the content of the active drug on the outer wall and the content of the active drug on the side walls are greater than the content of the active drug on the inner wall; or,
the active drug is only distributed on the outer wall and the side walls, and the inner wall does not contain the active drug.

2. The drug eluting device according to claim 1, wherein at least a portion of the drug coating on the inner wall has a pore structure.

3. The drug eluting device according to claim 2, wherein the pore structure has a porosity in a range of 5% to 60%.

4. The drug eluting device according to claim 1, wherein in the drug coating, the content of the active drug is in a range of 0.5 µg/mm² to 6 µg/mm².

5. The drug eluting device according to claim 1, wherein the drug carrier is a degradable polymer, and the degradable polymer is selected from at least one of a degradable polyester and a degradable polyanhydride.

6. The drug eluting device according to claim 1, wherein the drug coating has a thickness in a range of 2 microns to 20 microns.

7. The drug eluting device according to claim 1, wherein both of the thickness of a portion of the drug coating on the outer wall and the thickness of a portion of the drug coating on the side walls are greater than the thickness of the portion of the drug coating on the inner wall.

8. The drug eluting device according to claim 1, wherein the substrate comprises pure iron or iron alloy.

9. The drug eluting device according to claim 8, wherein the drug eluting device further comprises a zinc-containing protective layer, the zinc-containing protective layer is arranged on the substrate, the zinc-containing protective layer covers at least the inner wall of the substrate, and the zinc-containing protective layer is covered by the drug coating.

10. The drug eluting device according to claim 9, wherein the coverage of the zinc-containing protective layer on the substrate is in a range of 20%-100%.

11. A method of preparing a drug eluting device, comprising:
providing a substrate, the substrate having an outer wall, side walls, and an inner wall;
applying a solution containing an active drug and a drug carrier to the substrate to form a drug coating, wherein the active drug is dispersed in the drug carrier, and the drug coating covers the outer wall, the side walls, and the inner wall of the substrate; and
using a solvent to contact a portion of the drug coating on the inner wall such that the active drug at the portion of the drug coating on the inner wall is partially or completely dissolved in the solvent and the drug carrier is insoluble in the solvent, and removing the solvent from the substrate.

12. The method of claim 11, wherein using the solvent to contact the portion of the drug coating on the inner wall such that the active drug at the portion of the drug coating on the inner wall is partially or completely dissolved in the solvent and the drug carrier is insoluble in the solvent, and removing the solvent from the substrate comprises using an adsorbent material adsorbing the solvent to contact the inner wall of the substrate such that the active drug at the portion of the drug coating on the inner wall is partially or completely dissolved in the solvent and the drug carrier is insoluble in the solvent, and then removing the adsorbent material.

13. The method of claim 12, wherein the adsorbent material is a nylon material, polypropylene, a polytetrafluoroethylene material, a rubber-like material, or a fiber-like material.

14. The method of claim 12, wherein time of contact between the adsorbent material adsorbing the solvent and the inner wall of the substrate is in a range of 10 s to 20 min.

15. The method of claim 12, wherein using the adsorbent material adsorbing the solvent to contact the inner wall of the substrate such that the active drug at the portion of the drug coating on the inner wall is partially or completely dissolved in the solvent and the drug carrier is insoluble in the solvent, and then removing the adsorbent material comprises:
providing a core shaft, wrapping an outer surface of the core shaft with the adsorbent material, then putting the core shaft wrapped with the adsorbent material into an interior of the substrate, causing the adsorbent material to adsorb the solvent, using the adsorbent material to contact the inner wall of the substrate such that the active drug at the portion of the drug coating on the inner wall is partially or completely dissolved in the solvent, and then removing the adsorbent material and the core shaft from the substrate; or,
providing a core shaft, wrapping an outer surface of the core shaft with the adsorbent material, causing the adsorbent material to adsorb the solvent, then putting the core shaft wrapped with the adsorbent material into an interior of the substrate, using the adsorbent material to contact the inner wall of the substrate such that the active drug at the portion of the drug coating on the inner wall is partially or completely dissolved in the solvent, and then removing the adsorbent material and the core shaft from the substrate; or,
providing a core shaft made of the adsorbent material, causing the core shaft to adsorb the solvent, then putting the core shaft adsorbing the solvent into the interior of the substrate, using the core shaft to contact the inner wall of the substrate such that the active drug at the portion of the drug coating on the inner wall is partially or completely dissolved in the solvent, and then removing the core shaft from the substrate; or,
providing a core shaft made of the adsorbent material, putting the core shaft into the interior of the substrate, causing the core shaft to adsorb the solvent, using the core shaft to contact the inner wall of the substrate such that the active drug at the portion of the drug coating on the inner wall is partially or completely dissolved in the solvent, and then removing the core shaft from the substrate; or,
wiping the inner wall of the substrate with the absorbent material absorbing the solvent such that the active drug at the portion of the drug coating on the inner wall is partially or completely dissolved in the solvent, and then removing the adsorbent material from the substrate.

16. The method of claim 15, wherein when using the adsorbent material adsorbing the solvent to contact the inner wall of the substrate, the core shaft or the substrate is in a rotated state.

17. The method of claim 15, wherein after using the solvent to contact the portion of the drug coating on the inner wall such that the active drug at the portion of the drug coating on the inner wall is partially or completely dissolved in the solvent and the drug carrier is insoluble in the solvent, and removing the solvent from the substrate, the content of the active drug on the substrate is reduced by up to 50%.
